# EUROPÄISCHE PATENTANMELDUNG

(11) **EP 1 829 560 A1**
(43) Veröffentlichungstag der Anmeldung: **05.09.2007**
(21) Anmeldenummer: 07003401.2
(22) Anmeldetag: 19.02.2007
(51) Int. Cl.: A61L 9/12, A61L 9/14, B01D 1/18, G01N 33/14

(54) **Gerät zur Extraktion flüchtiger Bestandteile aus Flüssigkeiten**

(30) Priorität: 22.02.2006 DE 102006008200
(71) Anmelder: Sailer, Markus, 82110 Germering (DE); Klinger, Rudolf, 85567 Grafing (DE)
(72) Erfinder: Sailer, Markus, 82110 Germering (DE); Klinger, Rudolf, 85567 Grafing (DE)

(57) **Zusammenfassung**

Die Erfindung betrifft ein Gerät zur Extraktion flüchtiger Bestandteile aus Flüssigkeiten.

Die Erfindung stellt ein Zerstäubergerät zur Extraktion flüchtiger Bestandteile aus Flüssigkeiten, insbesondere bei der Lebensmitteltechnologie oder im Parfumwesen dar, womit flüchtige Substanzen aus einem flüssigen Substanzgemisch in der gasförmigen Phase angereichert zur Verfügung gestellt werden können. Dazu wird die Flüssigkeit (1) durch ein Förderrohr (2) aus einer Vorlage (3) gefördert und durch eine Zerstäuberdüse (4), die mit komprimiertem Gas über ein Druckrohr (5) betrieben wird, in einen Sprühnebel (8) verwandelt, der nach einem Kondensationsweg (9) auf eine Fläche (10) in der Kondensationskammer (11) geblasen wird, an der sich die Nebeltröpfchen niederschlagen und die schwerflüchtigen Bestandteile der Flüssigkeit kondensieren, während die leichtflüchtigen Bestandteile vermehrt in der Gasphase verbleiben. Die Gasphase steht daraufhin für Anwendungen oder Untersuchungen wie sensorische Beurteilungen zur Verfügung.

## Beschreibung

### Stand der Technik

Im Parfumwesen wird die Vergrößerung der Oberfläche bei der Zerstäubung von Flüssigkeiten angewandt, um flüssige Substanzen in die Gasphase zu überführen. Auch in der Oenologie, der Lebensmitteltechnologie und der Medizin wird die Vergrößerung der Oberfläche z.B. beim Schwenken von Weinen im Glas zur sensorischen Beurteilung, der Vernebelung von Aromasubstanzen oder das Feinverteilen von Substanzen zur Inhalation benutzt. Anlagen und Geräte zu diesem Zweck benutzen eigene Vorlagen und überführen die gesamte Flüssigkeit in die Gasphase bzw. in ein Aerosol und selektieren allenfalls nach der Größe der Nebeltropfen, nicht jedoch die Substanzen in der Lösung. Eine Verstärkung eines sensorischen, insbesondere olfaktischen Eindrucks sowie eine Komposition mehrerer Geruchsstoffe in der Gasphase war bisher nicht möglich.

### Probleme und Erfordernisse

Ein Problem bei der Wahrnehmung von Aromasubstanzen ist deren unterschiedliche Entfaltung in unterschiedlich geformten Gefäßen. Besonders stark kommt dies ins Bewußtsein, wenn man die Geruchs- und Geschmacksvarianz von Weinen aus unterschiedlich geformten Weingläsern betrachtet, was zu einer sehr hohen Anzahl an unterschiedlichen Weingläsern für die einzelnen Rebsorten geführt hat. Dennoch blieb bisher die sensorische Beurteilung von Weinen einem kleinen Kreis von Experten vorbehalten, deren Nase so gut geschult ist, daß sie die Komplexität der Aromastruktur erfassen können. Ziel unserer Entwicklung war es einerseits, das Geruchsbild eines Weines oder anderer Testsubstanzen so zu verstärken oder getrennt vom Gefäß zur Verfügung zu stellen, daß es auch für Personen mit durchschnittlicher Geruchssensibilität erfaßbar wird. Zudem sollten verschiedene Geruchsbereiche einzeln aus dem Gesamtbild herausgehoben werden, um näher begutachtbar zu werden. Um die Wahrnehmung nicht zu verfälschen war es nötig, ein Gerät zu konstruieren, das klein genug ist, um es variabel und ortsungebunden in unterschiedlichen Gefäßen wie Verkostungsgläsern einzusetzen. Zudem wäre eine schnelle und effektive Reinigung des Geräts für den Einsatz bei breiteren Verkostungen oder Tests wünschenswert.

Im Parfumwesen werden bei der Komposition unterschiedlicher Geruchsnoten teils sehr kostbare Öle verwendet, die durch ein Verschneiden mit Fremdsubstanzen wertlos werden. Bisher war es nicht möglich, die flüssigen Reinsubstanzen in ihrer ursprünglichen Form zu erhalten und nur die gasförmige Phase zu nutzen. Um die Reinsubstanzen zu erhalten, war es nötig, eine Vorrichtung zu konstruieren, bei der die parallel bei mehreren Aromastoffen aus der flüssigen in die Gasphase gebrachten Aromastoffe erst in der Gasphase vermengt werden.

Für wissenschaftliche und medizinische Anwendungen wie die Extraktion von luftempfindlichen flüchtigen Verbindungen ist es zudem nötig, die Extraktion der flüchtigen Stoffe auch mit Inertgasen wie z. B. Argon oder Stickstoff durchführen zu können, um einen Schutz der in Gasphase besonders anfälligen Verbindungen zu gewährleisten. Dazu mußten Druckzuleitungen gewählt werden, die den Anschluß an entsprechende Inertgasquellen möglich machen. Um die flüchtigen Stoffe in der Gasphase sammeln und untersuchen zu können, mußten zudem Schnittstellen geschaffen werden, um die flüchtigen Substanzen im Gasstrom über geeignete Anschlüsse in Sammel- oder Analyseapparaturen überführen zu können. Ein weiteres Ziel unserer Entwicklung war es somit, eine mit flüchtigen Substanzen beladene Gasphase für weitere Anwendungen bereitzustellen.

### Aufgabe und Basisfunktion der Erfindung

Es ist Aufgabe der Erfindung, eine mit flüchtigen Stoffen angereicherte Gasphase zur Verfügung zu stellen. Die flüchtigen Stoffe sollen durch einen Gasstrom aus einer Flüssigkeit extrahiert werden. Diese Aufgabe wird erfindungsgemäß durch den Gegenstand des Patentanspruchs 1 gelöst. Vorteilhafte Weiterbildungen der Erfindung sind Gegenstand der Unteransprüche.

Die Bereitstellung einer mit flüchtigen Stoffen stark angereicherten Gasphase kann durch
A. starke Oberflächenvergrößerung mittels Zerstäubung
B. und zeitnahe Kondensation der Flüssigkeit erreicht werden.
C. Ein vollständiger Übergang der Flüssigkeit in die Gasphase wird vermieden, indem der Sprühnebel (8) auf eine Oberfläche (10) geblasen wird, derart daß sich die Nebeltröpfchen abscheiden und in die Vorlage (3) zurückfließen, derart daß die flüchtigen Bestandteile der Flüssigkeit vermehrt in die Gasphase gelangen und zusammen mit dem Gasstrom aus der Düse (4) zum Auslaß (13) transportiert werden, während sich die Flüssigkeitsmenge der Vorlage (3) nur unwesentlich reduziert.

Das Volumen und die Form des Raumes, in dem die Kondensation geschieht, sind von entscheidender Bedeutung für die Zusammensetzung der extrahierten flüchtigen Stoffe. Diesem Umstand wird in der Fertigung mehrerer Ausführungen des Gerätes Rechnung getragen.

### Ausführungsformen

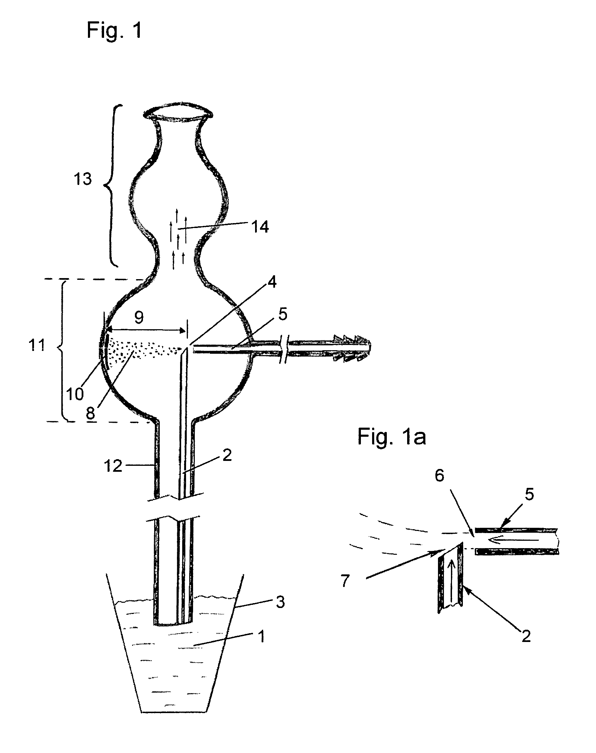

Eine vorteilhafte Ausführung (Fig. 1) ist eine weitgehend geschlossene Anordnung mit kleinem Gasauslaß (13), die über ein Druckrohr (5) seitlich mit komprimiertem Gas und über ein Förderrohr (2) mit Flüssigkeit (1) versorgt wird. Bei der geschlossenen Form wird der mit flüchtigen Substanzen angereicherte Gasstrom (14) über einen Auslaß (13) aus der Kondensationskammer (11) geführt. Die Ummantelung (12) zum Schutz des Förderrohres (2) dient zudem als Rücklauf für die kondensierte Flüssigkeit in die Vorlage (3). Die Größe der Kondensationskammer (11) ist dabei der zu testenden Substanz anzupassen. Durch diese Anordnung wird eine Beurteilung der Substanzen unabhängig von der Form der Vorlage (3) ermöglicht. Durch die Verwendung einer geeigneten Verbindung, wie Glasschliffe, Schraubverbindungen oder Ähnliches befestigte Übergangsstücke oder -schläuche, kann der Gasstrom (14) vom Auslaß (13) für weitere Anwendungen, Analysen oder Ähnliches zur Verfügung gestellt werden. Da der Gasstrom (14) zur weiteren Begutachtung aus dem Gerät herausgeführt wird und das Innere der Kammer nur schwer zugänglich ist, wird diese Ausführung im folgenden als geschlossene Kondensationskammer bezeichnet.

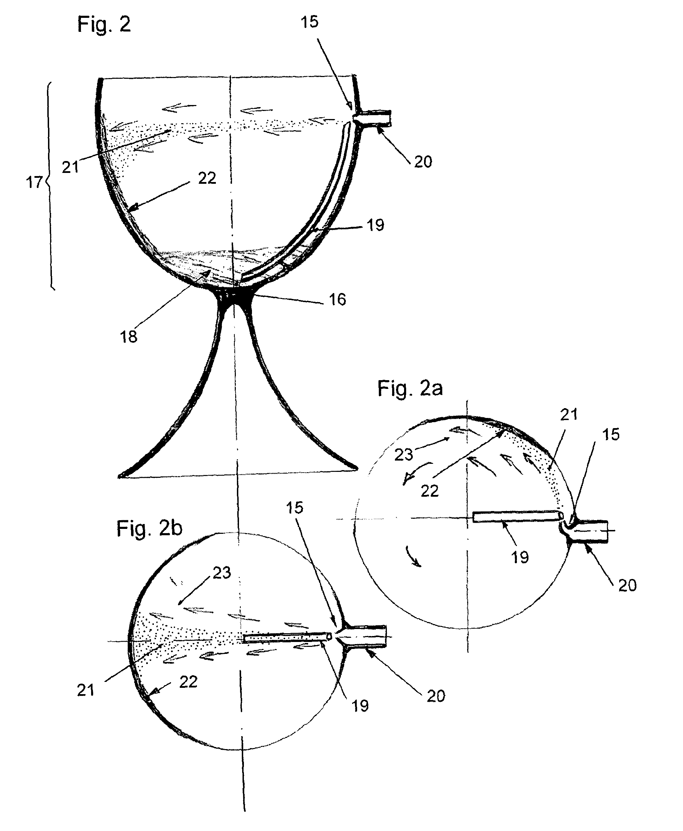

In einer weiteren vorteilhaften Ausführung (Fig. 2) wird die Kondensationskammer (17) in Größe und Form Testbehältern (16), wie etwa einem Weinglas, angepaßt. Sie dient damit auch als Vorlage für die Flüssigkeit (18). Die Druckzuleitung (20) für die Zerstäuberdüse (15) wird durch die Wandung geführt und der Sprühnebel (21) in einem Winkel auf die Wand (22) gerichtet, wodurch sich der Sprühnebel (21) niederschlägt und in den Gefäßgrund rückläuft. Durch einen Flachen Auftreffwinkel (Fig. 2a) wird eine kreisende Bewegung des Gasstromes (23) im Gefäß induziert, während eine gerade Sprührichtung senkrecht auf die Wand (Fig. 2b) eine maximale Verwirbelung des Gasstromes (23) bewirkt. Das mit flüchtigen Substanzen beladene Gas (23) verbleibt in der Kondensationskammer (17) und steht dort zur Verfügung. Da die Tests des Gases (23) mit der im Inneren des Gefäßes verbliebenen Gasmenge durchgeführt werden und somit das Innere der Kammer für die Prüfung zugänglich sein muß, wird im folgenden diese Art der Ausführung als offene Kondensationskammer bezeichnet.

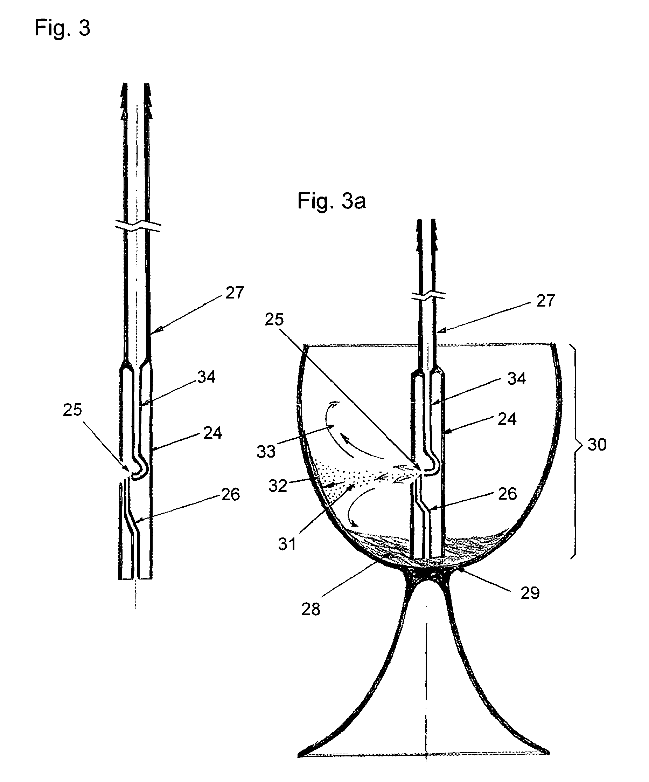

Eine weitere vorteilhafte Ausführung (Fig. 3) besteht aus einer mobilen separaten und durch ein Gehäuse (24) geschützten Verneblereinheit mit kurzem Förderrohr (26) und als Griff ausgebildeter Druckzuleitung (27), die bevorzugt linear angeordnet sind. Dadurch kann der Vernebler gut in Probengefäße (29) wie etwa Wein- oder Verkostungsgläser eingeführt und mit dem Förderrohr (26) in die Flüssigkeit (28) eingetaucht werden (Fig. 3a). Die Düse (25) erzeugt einen Sprühnebel (31), der senkrecht zu der Achse Druckzuleitung (27) - Förderrohr (26) austritt und sich an der Wandung (32) des Vorlagegefäßes niederschlägt. Die Probengefäße (29) dienen dabei als Vorlage und offene Kondensationskammer (30), die Wandung dient als Kondensationsfläche (32). Der mit flüchtigen Substanzen beladene Gasstrom (33) verbleibt im Probengefäß (29), während das Zerstäubergerät nach der Anwendung entfernt werden kann. Das Gerät kann in dieser Ausführung sehr klein und handlich gestaltet werden und eignet sich damit besonders zum ortsungebundenen Einsatz. Der Betrieb des Gerätes kann sehr einfach über ein Handgebläse oder einen Blasebalg mit Druckluft erfolgen.

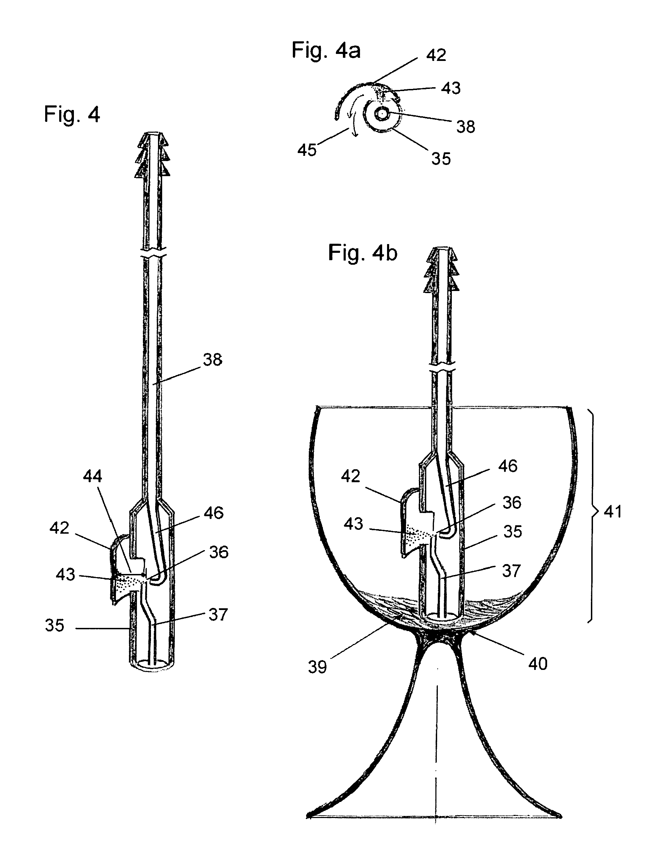

Eine bevorzugte Ausführung (Fig. 4) besteht aus einer mobilen separaten und durch ein Gehäuse (35) geschützten Verneblereinheit mit kurzem Förderrohr (37) und als Griff ausgebildeter Druckzuleitung (38), die bevorzugt linear angeordnet sind. Dadurch kann der Vernebler gut in Probengefäße (40) wie etwa Wein- oder Verkostungsgläser eingeführt und mit dem Förderrohr (37) in die Flüssigkeit (39) eingetaucht werden (Fig. 4b). Die Düse (36) erzeugt einen Sprühnebel (43), der senkrecht zu der Achse Druckzuleitung (38) - Förderrohr (37) austritt und an einer Kondensationsfläche (42), die sich mantelförmig um das Gehäuse (35) der Verneblereinheit legt, kondensiert. Dadurch wird ein Sprühnebelfleck an der Wandung des Probengefäßes (40) vermieden und gleichzeitig ein konstanter Nebelweg (44) geschaffen. Durch die Ablenkung (Fig. 4a) des Gasstromes (45) an der Kondensationsfläche (42) wird das Gas (45) im Probengefäß zum Kreisen innerhalb des Gefäßes gebracht, wo es verbleibt, während das Zerstäubergerät nach der Anwendung entfernt werden kann. Das Gerät kann in dieser Ausführung sehr klein und handlich gestaltet werden und eignet sich damit besonders zum ortsungebundenen Einsatz. Der Betrieb des Gerätes kann sehr einfach über ein Handgebläse oder einen Blasebalg mit Druckluft erfolgen.

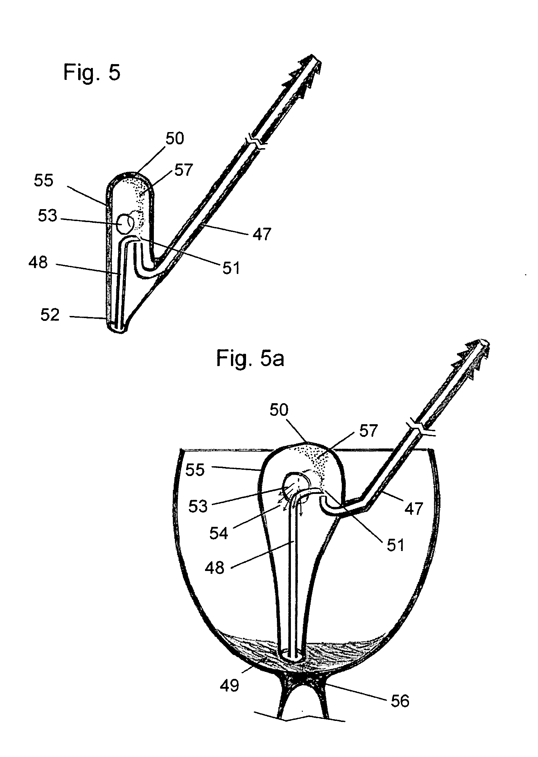

In einer weiteren Ausführung (Fig. 5) besitzt der Vernebler ein kurzes Förderrohr (48) und eine als Griff ausgebildete Druckzuleitung (47). So kann der Zerstäuber gut in Probengefäße (56) wie Verkostungsgläser eingeführt und mit dem Förderrohr (48) in die Flüssigkeit (49) eingetaucht werden (Fig. 5a). Die Düse (51) ist von einer Kondensationskammer (55) umgeben, aus der nach der Kondensation der Flüssigkeit an der Kondensationsfläche (50) der mit flüchtigen Stoffen angereicherte Gasstrom (54) durch einen Auslaß (53) entweicht, während das Kondensat parallel zum Förderrohr (48) abläuft. Durch diese Anordnung können durch eine Variation der Kondensationskammer (55) in Volumen und Form unterschiedliche Geruchskomponenten hervorgehoben werden. Der Betrieb des Gerätes kann ortsungebunden sehr einfach über ein Handgebläse oder einen Blasebalg mit Druckluft erfolgen.

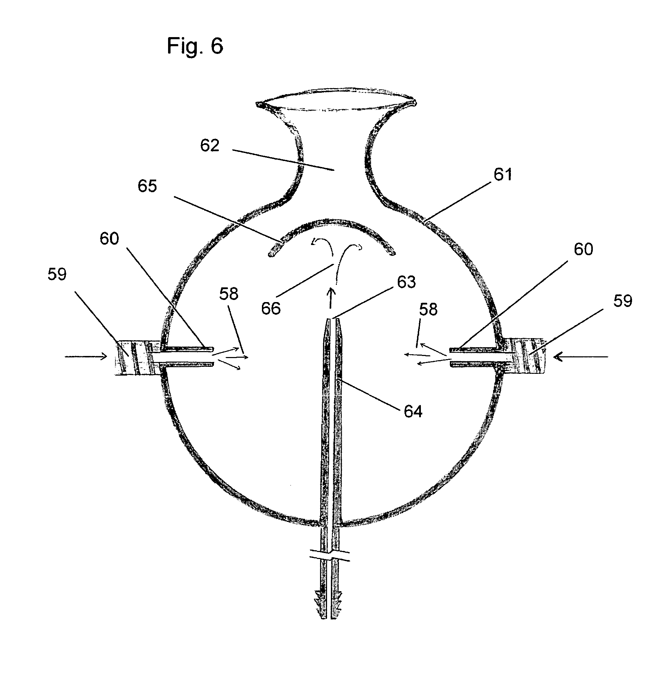

Für den parallelen Betrieb mehrerer Vernebler ist eine weitere Ausführung (Fig. 6) vorteilhaft. Durch eine Modifikation der Auslässe (13) mit geeigneten Verbindungen können die mit flüchtigen Stoffen beladenen Gasströme (58) von mehreren Verneblereinheiten durch Anschlüsse (59) über Einlaßöffnungen (60) in eine Mischkammer (61) geleitet und dort vereinigt werden. Vorteilhaft ist hierzu eine regelbare Druckzufuhr mit komprimiertem Gas. Aus der Mischkammer (61) können die gemischten Fraktionen ggf. durch einen angelegten Gasstrom (66) durch den Auslaß (62) ausgeblasen werden. Dazu wird über eine Druckleitung (64) und eine Düse (63) ein Gasstrom (66) auf ein Schild (65) geblasen, wodurch die Gase im inneren der Mischkammer (61) intensiv vermischt werden. Diese Anordnung ist besonders vorteilhaft zum kontrollierten Vermischen von Gasströmen, die mit flüchtigen Substanzen wie Aromaölen beladen sind oder um einem Gasstrom (58) eine bekannte Komponente in Form von einem weiteren Gasstrom als Standard zuzusetzen. Durch eine Modifikation des Auslasses (62) aus der Mischkammer (61) können der Gasstrom für weitere Anwendungen verwendet oder die Substanzen gesammelt werden.

### Werkstoffe

Als Werkstoff wurde für alle Geräte Glas gewählt, das wegen seiner guten Werkstoffeigenschaften und Inertheit große Vorteile besitzt. Grundsätzlich sind jedoch auch andere Werkstoffe, die keine Wechselwirkungen mit den Substraten zeigen, wie Stahl, Kunststoffe, Keramik oder Ähnliches, denkbar.

Im folgenden wird die Funktionsweise der Erfindung anhand von Zeichnungen der unterschiedlichen Ausführungsformen beschrieben. Die Zeichnungen zeigen
Fig. 1 einen erfindungsgemäßen Zerstäuber mit Kondensationskammer in Schnittdarstellung und Vergrößerung der Zerstäuberdüse (1a),
Fig. 2 einen erfindungsgemäßen Zerstäuber mit integrierter Vorlage in Form eines Weinglases in Schnittdarstellung, Aufsicht mit flachem Düsenwinkel (2a) und Aufsicht mit steilem Düsenwinkel (2b),
Fig. 3 einen Zerstäuber für die Verwendung von Probengefäßen als Vorlage und Kondensationskammer in Schnittdarstellung und im Gefäß (3a),
Fig. 4 einen Zerstäuber ummantelt mit Kondensationsfläche für die Verwendung in Probengefäßen in Schnittdarstellung, in der Aufsicht (4a) und im Gefäß (4b),
Fig. 5 einen Zerstäuber mit Kondensationskammer zur Aromenselektion in kleiner Ausführung und großer Ausführung im Glas (5a),
Fig. 6 eine Mischkammer.

Die Merkmale der Geräte werden mit arabischen Zahlen beziffert.

### Genaue Funktionsbeschreibung

Das Gerät zur Anreicherung flüchtiger Substanzen in der Gasphase arbeitet mit einer Düse (4), die nach dem bekannten Bernoulli-Prinzip funktioniert. Dabei wird an der der Austrittsöffnung (6) des Druckrohres (5) abgewandten und abfallenden Seite des Förderrohres (7) ein Unterdruck erzeugt, durch den die Flüssigkeit (1) durch das Förderrohr (2) angesaugt wird. Die angesaugte Flüssigkeit (1) wird im Gasstrom, der die Austrittsöffnung (6) des Druckrohres (5) verläßt, in Form von feinsten Tröpfchen mitgerissen und vernebelt (8). Durch die Ausrichtung der Düse (4) wird die vernebelte Flüssigkeit (8) zur Kondensation auf eine Fläche (10) geblasen, an der sich die Flüssigkeit abscheidet. Durch die Entfernung der Kondensationsfläche (10) von der Düse (4) ist der Nebelweg (9) vorgegeben. Da die flüchtigen Substanzen aus der Flüssigkeit (1) in der Zeit, in der die Flüssigkeit (1) feinverteilt im Gasstrom vorliegt, besonders gut aus der flüssigen Phase in die gasförmige Phase übergehen können, ist die Länge des Nebelweges (9) und damit die Verweilzeit der Flüssigkeit (1) als Nebel (8) für den Übergang der flüchtigen Stoffe von der flüssigen Phase in die Gasphase von entscheidender Bedeutung und muß den jeweiligen Eigenschaften der Substanzen angepaßt werden. Ebenso sind der Winkel, in dem der Nebel (8) auf die Kondensationsfläche (10) geblasen wird und die Beschaffenheit der Kondensationsfläche (10) von großer Bedeutung für die Ausprägung des Übergangs flüchtiger Substanzen in die Gasphase. Diese Zusammenhänge machen eine variable Gestaltung der Kondensationskammer (11) zwingend notwendig. Der Gasstrom (14) aus dem Druckrohr (5) ist nach der Kondensation der Flüssigkeit an der Kondensationsfläche (10) mit flüchtigen Substanzen beladen. Er wird durch einen Auslaß (13), der möglichst orthogonal zur Sprühnebelrichtung angeordnet ist, aus der Kondensationskammer (11) geführt, dann gegebenenfalls unter Zuhilfenahme von bekannten Applikationen wie Kugeln oder Vigreuxaufsätzen gereinigt und zur Verfügung gestellt.

Die Geräte können mit geschlossener Kondensationskammer (Fig. 1; 5), bei der die Düse (4) in der Kondensationskammer (11; 55) fixiert ist und der Gasstrom durch einen relativ kleinen Auslaß (13; 53) abgeleitet wird, und mit offener Kondensationskammer (Fig, 2; 3; 4), bei der das beladene Gas aus dem Druckrohr in der Kondensationskammer (17; 30; 41) verbleibt, genutzt werden. Bei geschlossener Kondensationskammer geschieht die Prüfung der Substanzen im Gasstrom außerhalb der Kammer, bei der offenen Kondensationskammer kann die Prüfung im Inneren der Kammer geschehen. Um Gefäße wie Wein- oder Verkostungsgläser gleichzeitig als Vorlage und Kondensationskammer nutzen zu können (Fig. 3; 4), ist das Zerstäubergerät mit einem Schutzgehäuse versehen und das Druckzuleitungsrohr mit dem Förderrohr in eine Achse gebracht, um eine platzsparende und handliche Apparatur zu erhalten. Ein unschöner Niederschlag des Sprühnebels (31) an der Glaswand kann durch das Anbringen einer Kondensationsfläche (42) am Schutzgehäuse (35) vermieden werden, wodurch auch der Nebelweg (44) normiert wird. Alle Tauchgeräte (Fig. 3; 4; 5) sind zum Einsatz in offenen Glasgefäßen prädestiniert, etwa für Verkostungen. Dabei spielen auch kleine Nebelreste, die vom Gasstrom nach der Kondensation mitgerissen werden, keine Rolle; sie schlagen sich im Gefäßgrund nieder oder verdunsten, noch bevor sie einen negativen sensorischen Eindruck hinterlassen können. Die Geräte, die zu Verkostungen eingesetzt werden, sind mit Druckluft durch Handgebläse zu bedienen. Dafür werden die Geräte innerhalb der Gefäße in die dort vorgelegten Substanzen eingetaucht und durch kurze Pumpstöße bedient. Nach dem Entfernen des Gerätes aus dem Gefäß kann der mit Aromastoffen angereicherte Gasinhalt des Gefäßes sensorisch beurteilt werden.

Geräte mit geschlossener Kondensationskammer (Fig. 1; 5) können einerseits dazu benutzt werden, den substanzbeladenen Gasstrom räumlich unabhängig von der Vorlage zur Verfügung zu stellen, andererseits können durch Größe und Form der Kammer bestimmte flüchtige Substanzen bevorzugt in die Gasphase gebracht werden. Besonders für Verkostungen kann eine selektive Hervorhebung unterschiedlicher Geruchsbereiche hilfreich sein; so werden durch kleine kugelige Kondensationskammern (55) (Fig. 5) frische Zitrusnoten verstärkt hervorgehoben, während große tropfenförmige Kondensationskammern (55) (Fig. 5a) verstärkt Strukturnoten fördern. Für schlecht zugängliche Vorlagen wie Flaschen ist es hilfreich, wenn die flüchtigen Substanzen an anderer Stelle unabhängig von der Vorlage zur Verfügung gestellt werden. Durch ein Gerät, wie in Fig. 1 dargestellt, kann dies geschehen; auch kann durch diese Ausführung in Verbindung mit geeigneten Anschlüssen und Leitungen das Trägergas für weitere Anwendungen zur Verfügung gestellt werden.

Besonders zur Komposition von beladenen Gasströmen aus unterschiedlichen Reinextrakten, ätherischen Ölen oder anderen Substanzen, die aus unterschiedlichen parallel betriebenen Geräten zur Verfügung gestellt werden, ist eine Mischkammer (61) nötig. Dabei werden die Gasströme (58) über geeignete Verbindungen und durch Anschlüsse (59) und Einlaßöffnungen (60) in die Mischkammer (61) geleitet. Die Gasströme (58) werden in der Kammer (61) durch die Bewegung der einzelnen Gasströme vermengt und durch die Auslaßöffnung (62) entlassen. Im Falle von zu geringer Strömung kann über ein Druckrohr (64) durch eine Düse (63) zusätzlich ein Gasstrom (66) angelegt werden, der durch ein Schild (65) zusätzlich verwirbelt wird und so die Durchmischung der Gasströme in der Kammer fördert. An der Auslaßöffnung (62) kann die sensorische Beurteilung oder die weitere Verarbeitung der verflüchtigten Substanzen in Form von Kollektoren oder Analysegeräten erfolgen.

### Vergleichspatente:

EP 0261649 B2
EP 0540774 B1
EP 0923985 B1
EP 1023911 A2
DE 9315778 U1
DE 10146283 A1
DE 20209725 U1
DE 19925273 A1
DE 19909218 A1
DE 19903374 C2
DE 19755643 C2
DE 10002005 A1
DE 811264 C

### Bezugszeichenliste

- Fig. 1:: Zerstäubergerät Basismodell
- Fig. 1a:: Vergrößerung der Zerstäuberdüse
- Fig. 2:: Zerstäuber, fest im Probengefäß verbaut (Am Beispiel Weinglas)
- Fig. 2a:: Aufsicht für stumpfen Sprühwinkel schräg an die Gefäßwand
- Fig. 2b:: Aufsicht für rechten Sprühwinkel senkrecht an die Gefäßwand
- Fig. 3:: Zerstäubergerät zum eintauchen in Probegefäße
- Fig. 3a:: Anwendung des Tauchgerätes im Glas
- Fig. 4:: Tauchzerstäuber mit Kondensationsfläche zum eintauchen in Probegefäße
- Fig. 4a:: Zeichnung in der Aufsicht
- Fig. 4b:: Anwendung des Tauchgerätes mit Kondensationsfläche im Glas
- Fig. 5:: Tauchzerstäuber zur Aromenselektion, Beispiel für kleines Gerät
- Fig. 5a:: Tauchzerstäuber zur Aromenselektion in Anwendung, Beispiel für große Ausführung.
- Fig. 6:: Mischkammer
- Fig. 1:: 1 Flüssigkeit
2 Förderrohr
3 Vorlage
4 Zerstäuberdüse
5 Druckrohr
6 Austrittsöffnung des Druckrohres
7 Förderrohröffnung, angeschrägt
8 Sprühnebel
9 Kondensationsweg
10 Kondensationsfläche
11 Kondensationskammer
12 Förderrohrschutz
13 Auslaß
14 Gasstrom, mit flüchtigen Substanzen angereichert
- Fig. 2:: 15 Zerstäuberdüse
16 Gefäß
17 Kondensationskammer
18 Flüssigkeit
19 Förderrohr
20 Druckrohr
21 Sprühnebel
22 Kondensationsfläche
23 Gasstrom, mit flüchtigen Substanzen angereichert
- Fig. 3:: 24 Gehäuse für Düse
25 Zerstäuberdüse
26 Förderrohr
27 Druckrohr, als Griff verwendbar
28 Flüssigkeit
29 Gefäß
30 Kondensationskammer
31 Sprühnebel
32 Gefäßwand
33 Gasstrom, mit flüchtigen Substanzen angereichert
34 Druckrohr
- Fig. 4: 35 Gehäuse für Düse
36 Zerstäuberdüse
37 Förderrohr
38 Druckrohr, als Griff verwendbar
39 Flüssigkeit
40 Gefäß
41 Kondensationskammer
42 Kondensationsfläche
43 Sprühnebel
44 Kondensationsweg
45 Gasstrom, mit flüchtigen Substanzen angereichert
46 Druckrohr
- Fig. 5: 47 Druckrohr, als Griff verwendbar
48 Förderrohr
49 Flüssigkeit
50 Kondensationsfläche
51 Zerstäuberdüse
52 Förderrohrschutz
53 Auslaß
54 Gasstrom, mit flüchtigen Substanzen angereichert
55 Kondensationskammer
56 Gefäß
57 Sprühnebel
- Fig. 6: 58 Gasströme aus untersch. Zerstäubergeräten
59 Anschluß
60 Einlaßöffnung für Gasstrom
61 Mischkammer
62 Auslaßöffnung der Mischkammer
63 Düse zur Einleitung von Gasen
64 Druckrohr
65 Schild
66 Gasstrom

## Patentansprüche

1. Zerstäubergerät zur Extraktion flüchtiger Bestandteile aus Flüssigkeiten, insbesondere bei der Lebensmitteltechnologie oder im Parfumwesen, womit flüchtige Substanzen aus einem flüssigen Substanzgemisch in der gasförmigen Phase angereichert zur Verfügung gestellt werden können, wozu die Flüssigkeit (1) durch ein Förderrohr (2) aus einer Vorlage (3) gefördert und durch eine Zerstäuberdüse (4), die mit dem Bernoulli-Effekt arbeitet und mit komprimiertem Gas über ein Druckrohr (5) betrieben wird, in einen Sprühnebel (8) verwandelt wird, der nach einem Kondensationsweg (9) in einem Winkel auf eine Fläche (10) in der Kondensationskammer (11) geblasen wird, an der sich die Nebeltröpfchen niederschlagen und die schwerflüchtigen Bestandteile der Flüssigkeit kondensieren, während die leichtflüchtigen Bestandteile vermehrt in der Gasphase verbleiben.

2. Zerstäubergerät nach Patentanspruch 1
**dadurch gekennzeichnet,**
**daß** das Förderrohr (2) in die in der Vorlage (3) befindlichen Flüssigkeit (1) eingetaucht ist und daß die Flüssigkeit über das Förderrohr zur Zerstäuberdüse (4) transportiert werden kann.

3. Zerstäubergerät nach Patentanspruch 1 oder 2
**dadurch gekennzeichnet,**
**daß** das Förderrohr (2) der Viskosität der Flüssigkeit (1) angepaßt ist, daß das Förderrohr (2) aus dem Gerät herausgezogen ist, daß das Förderrohr (2) in die Kondensationskammer (11) eintritt und von einem Schutzrohr (12) umgeben ist, und daß das Schutzrohr gleichzeitig als Rücklauf für die kondensierte Flüssigkeit zurück in die Vorlage (3) dient, und mit der Kondensationskammer (11) über eine Öffnung verbunden ist.

4. Zerstäubergerät nach einem der vorhergehenden Patentansprüche
**dadurch gekennzeichnet,**
**daß** der Auslaß (13) für den mit flüchtigen Substanzen angereicherten Gasstrom (14) mit Aufsätzen und Anschlüssen zur Aufbereitung und Weiterleitung des Gasstromes versehen ist.

5. Zerstäubergerät nach einem der vorhergehenden Patentansprüche
**dadurch gekennzeichnet,**
**daß** das Druckrohr (5) zum Betrieb der Düse (4) für den Anschluß einer geregelten Quelle von komprimiertem Gas oder Druckluft aus dem Gerät herausgeführt ist.

6. Zerstäubergerät nach einem der vorhergehenden Patentansprüche
**dadurch gekennzeichnet,**
**daß** die Kondensationsfläche (10) am Auftreffbereich des Sprühnebels (8) den Eigenschaften der Flüssigkeit (1) und der zu verflüchtigenden Flüssigkeitsbestandteile angepaßt ist, indem sie flach, konkav, konvex, trichterförmig erweitert oder zulaufend ausgebildet ist.

7. Zerstäubergerät nach einem der vorhergehenden Patentansprüche
**dadurch gekennzeichnet,**
**daß** die Kondensationskammer (11) in Form und Volumen den Eigenschaften der Flüssigkeit (1) und der zu verflüchtigenden Flüssigkeitsbestandteile angepaßt ist.

8. Zerstäubergerät nach einem der vorhergehenden Patentansprüche
**dadurch gekennzeichnet,**
**daß** die Zerstäuberdüse (15) in ein Gefäß (16) eingearbeitet ist, das als Vorlage und Kondensationskammer (17) fungiert, wobei die Flüssigkeit (18) über ein Förderrohr (19) aus dem Gefäßgrund gefördert, der Druckluftkanal (20) durch die Wandung des Gefäßes geführt und der Sprühnebel (21) durch die Düse (15) in einem Winkel an die Gefäßwand (22) geblasen wird und dort kondensiert, während die flüchtigen Bestandteile angereichert im Gasstrom (23) im Gefäß verbleiben.

9. Zerstäubergerät nach einem der vorhergehenden Patentansprüche
**dadurch gekennzeichnet,**
**daß** die durch ein Gehäuse (24) geschützte Zerstäuberdüse (25) aufgrund eines kurzen Förderrohres (26) mit Hilfe eines als Griff fungierenden Druckzuleitungsrohres (27) in ein mit Flüssigkeit (28) befülltes Gefäß (29) (z.B. Weinglas) eingeführt und mit kurzen Druckluftstößen aus einem Blasebalg betrieben wird, so daß das Gefäß (29) als Vorlage und offene Kondensationskammer (30) genutzt wird, der Sprühnebel (31) an der Gefäßwand (32) abläuft und die Gasphase innerhalb des Gefäßes (33) mit flüchtigen Stoffen angereichert wird.

10. Zerstäubergerät nach einem der vorhergehenden Patentansprüche
**dadurch gekennzeichnet,**
**daß** das Förderrohr (26) bzw. das Gehäuse (24) und das als Griff fungierende Druckzuleitungsrohr (27) in einer Achse angeordnet sind.

11. Zerstäubergerät nach einem der vorhergehenden Patentansprüche
**dadurch gekennzeichnet,**
**daß** die durch ein Gehäuse (35) geschützte Zerstäuberdüse (36) wegen ihres kurzen Förderrohres (37) mit Hilfe eines als Griff fungierenden Druckzuleitungsrohres (38) in ein mit Flüssigkeit (39) befülltes Gefäß (40) eingeführt und mit kurzen Druckluftstößen aus einem Blasebalg betrieben wird, so daß das Gefäß (40) als offene Kondensationskammer (41) genutzt wird, und daß konisch um die Zerstäuberdüse (36) eine Kondensationsfläche (42) angeordnet ist, an der der Sprühnebel (43) kondensiert, wodurch ein definierter Kondensationsweg (44) gegeben ist, und der Gasstrom (45) innerhalb des Gefäßes (40) auf eine Kreisbahn gezwungen wird, wodurch Kondensationstropfen an der Wandung des Gefäßes vermieden werden und die Gasphase innerhalb des Gefäßes mit flüchtigen Stoffen angereichert wird.

12. Zerstäubergerät nach einem der vorhergehenden Patentansprüche
**dadurch gekennzeichnet,**
**daß** das Zerstäubergerät eine geschlossene Einheit mit Druckluftzuleitungsrohr (47), das auch als Griff fungiert, ist, mit kurzem Förderrohr (48) für die Testflüssigkeit (49), mit einer auf eine Kondensationsfläche (50) gerichteten Zerstäuberdüse (51), mit einem Förderrohrschutz (52) als Rücklauf für die kondensierte Flüssigkeit, mit mindestens einem Auslaß (53) für den mit flüchtigen Bestandteilen angereicherten Gasstrom (54) und mit definierter Kondensationskammer (55), durch deren Volumen und Form die Zusammensetzung der zu extrahierenden flüchtigen Substanzen gesteuert wird, die in ein Gefäß (56) eingeführt ist und durch kurze Druckluftstöße betrieben wird.

13. Zerstäubergerät nach einem der vorhergehenden Patentansprüche
**dadurch gekennzeichnet,**
**daß** mehrere Zerstäubereinheiten mit getrennten Flüssigkeitsvorlagen und separaten Druckgaszuleitungen parallel und unabhängig voneinander betrieben werden, wobei die mit flüchtigen Substanzen beladenen Gasströme (58) aus den Auslaßöffnungen der Zerstäuber über angepaßte Verbindungen (59) und Einlaßöffnungen (60) in eine Mischkammer (61) überführt, vereinigt und verwirbelt werden und von dort gemeinsam durch eine Auslaßöffnung (62) ausgeblasen werden, wodurch definierte Mischungen an flüchtigen Substanzen hergestellt bzw. Standards für Vergleiche beigemengt werden können, ohne die ursprünglichen Flüssigkeiten zu vereinigen.

14. Zerstäubergerät nach einem der vorhergehenden Patentansprüche
**dadurch gekennzeichnet,**
**daß** die Mischkammer (61) mit einer Düse (63) versehen ist, deren Druckzuleitungsrohr (64) zur kontrollierten Versorgung mit einem komprimierten Gase herausgezogen ist und die auf ein Schild (65) gerichtet ist, um maximale Verwirbelung zu gewährleisten, mit deren Hilfe ein Gasstrom (66) angelegt werden kann, durch den die mit flüchtigen Substanzen beladenen Gase aus den Zerstäubereinheiten vermischt und zum Auslaß (62) transportiert werden.
